# EUROPEAN PATENT APPLICATION

(11) **EP 2 633 852 A1**
(43) Date of publication of application: **04.09.2013**
(21) Application number: 13157381.8
(22) Date of filing: 01.03.2013
(51) Int. Cl.: A61K 9/00, A61K 9/08, A61K 47/42, A61K 8/00, A61K 47/44

(54) **Jojoba oil-containing ophthalmic spray composition**

(30) Priority: 01.03.2012 IT FI20120044
(71) Applicant: Biodue S.p.A., 50028 Tavarnelle Val di Pesa (IT)
(72) Inventor: Benedetti, Ruffo, 50023 Impruneta (IT)
(74) Representative: Brighenti, Livio

(57) **Abstract**

Described herein is an ophthalmic spray, comprising liposomes containing jojoba oil, that is useful for treating MGD in particular.

## Description

### Field of the invention

The present invention relates to the field of formulations for ophthalmic use.

### Background of the invention

As is known, humidity control is essential for cellular function in general as it allows our skin to be maintained soft, smooth and flexible however, it is in the eyes that it plays a decisive role in that, in addition to the above-mentioned function, it provides for maintaining clarity of the cornea, which is essential for a good vision. A material similar to sebum, which is secreted by the modified sebaceous glands present in the eyelids, called meibomian glands, also provides for controlling the humidity of the eye.

This sebum contains more than 50% wax esters and forms a thin lipid layer on the tear film thus helping to prevent the loss thereof by evaporation.

Recent studies have demonstrated that when the so-called meibomian sebum is insufficient, tears evaporate four times faster.

The Meibomian glands can be subject to a hyperkeratinisation of the ductal epithelium therein, said MGD, which leads to an increased intra-ductal pressure and to stagnation of the normal flow of the meibomian gland. Obstruction of the meibomian ducts causes accumulation and thickening of the lipids, bacterial colonisation and inflammation of the eyelid margins. Secondary effects include a compromised tear film with associated dry eye syndrome.

MGD currently represents one of the most neglected aspects of ocular comfort and adequate treatment methods have yet to be found.

Given the difficulty of restoring the normal chemistry, texture and flow of the meibomian sebum, integrating the tear film with a similar substance would be a good way to decrease friction of the ocular surface and reduce inflammation of the ocular surface.

### Summary of the invention

An ophthalmic spray comprising liposomes containing jojoba oil, which is useful for topical ophthalmic topical, is described.

### Detailed description of the invention

The present invention allows the above-mentioned problems to be overcome thanks to a spray formulation comprising liposomes containing jojoba oil.

As is known, the so-called jojoba oil is actually a wax that melts at around 6°C, and is thus liquid at room temperature, which consists of long-chain monoesters; the similarity between jojoba oil wax esters and meibomian sebum wax esters, as well the safety of the use thereof on the eye, indicate this oil as the ideal replacement of meibomian sebum.

It is also known that liposomes are structures consisting of lipid bilayers that close forming small vesicles similar to the body's cells and the granules thereof, said vesicles are ideal carriers for transporting both hydrophilic substances (contained in the aqueous compartments thereof) and lipophilic substances (contained within the lipid bilayers) towards target organs.

It has now been surprisingly found that the liposomes according to the present invention, prepared according to known techniques and loaded with jojoba oil, are able to adhere to the corneal epithelium and to gradually release the active substance.

In particular, according to the invention, liposomes consist of phospholipids or cholesterol in which the jojoba oil (hydrophobic) is contained within the lipid bilayers.

The liposomes according to the present invention thus comprise jojoba oil and phospholipids (such as for example lecithin) and other components commonly used in analogous compositions such as: humectants/solvents, binders/stabilisers, rheological additives, osmotic concentration regulators, preservatives, chelating agents.

Sodium hydroxymethylglycinate and disodium EDTA are preferably selected as stabilisers and, in addition, a neutral pH (about 7) is to be maintained.

The liposomes according to the invention can also be utilised as a base for providing other active ingredients such as: N-acetyl-cysteine, eledoisin, hydroxy carotenoids, nervonic acid, coenzyme Q10, vitamin A and E, hyaluronic acid, urea, zinc, lactoferrin, antimicrobial peptides, lysozyme and other substances having immunomodulatory action, anti-inflammatory action or action stimulating eyelash growth.

For example, the liposomes according to the present invention can consist of the following components in the percentages indicated (by weight on the total weight of the finished product):

| | |
|---|---|
| Jojoba oil | 0.1 - 2%; |
| Butylene glycol | 0.25 - 2.75%; |
| Phospholipids | 0.04 - 0.44%; |
| Carrageenans | 0.0005 - 0.0055%; |
| Carbomer | 0.0028 - 0.0308%; |
| Sodium chloride | 0.75 - 0.85%; |
| Sodium hydroxymethylglycinate | 0.10 - 0.12%; |
| Disodium EDTA | 0.08 - 0.12; |
| Lactic acid | q.s. at pH = 6.5 -7.5; |
| Distilled water | q.s. at 100% w/v |

More in particular the liposomes according to the invention consist of:

| | |
|---|---|
| jojoba oil | 0.2%; |
| butylene glycol | 0.5%; |
| phospholipids | 0.08%; |
| carrageenans | 0.001%; |
| carbomer | 0.0056%; |
| sodium chloride | 0.8%; |
| sodium hydroxymethylglycinate | 0.11%; |
| disodium EDTA | 0.1%; |
| Lactic acid | q.s. at pH = 7; |
| distilled water | q.s. at 100% w/ |

Preferably the liposomes according to the invention have sizes of between 0.5 -1.5 µm.

The liposomes according to the invention are prepared according to well-known methods, by applying micro-fluidification techniques, and are formed by self-aggregation of the phospholipids in an aqueous phase in which the amphiphilic molecules of the phospholipids spontaneously give rise to spherical vesicles (bilayers) in an attempt to protect (segregate) the hydrophobic part of the molecule thereof from the aqueous environment, while the polar part remains in contact with the aqueous phase.

The liposome preparation method comprises 3 steps: hydration of the phospholipids, sizing of the liposomes and removal of the non-encapsulated material.

Hydration takes place by mechanical method: a thin lipid film is deposited by a solution in organic solvent onto the wall of a glass flask (rotary evaporator). The film is hydrated under stirring at a temperature higher than the critical temperature of the phospholipid having the highest critical temperature. Thus plurilamellar vesicles (PML) with wide dimensional distribution (greater than 0.1 µm) are thus obtained.

The homogenisation method is utilised for the sizing and the dimensional distribution of the liposomes: the dimensional distribution curve is narrowed by passing the liposome dispersion through a high pressure homogeniser. A bimodal dimensional distribution (with two frequency maximums), with particles of about 1 µm, is thus found

The removal of the non-encapsulated material is not necessary in that the jojoba oil is lipophilic, shows high affinity with the liposome bilayer and completely associates therewith.

The liposomes as above-obtained are then formulated in spray form, a pharmaceutical form which, in the ophthalmic field, distinguishes itself for its practicality of use.

The process for preparing said composition is also carried out using known techniques and provides for the following steps:
- introducing the distilled water into the mixer;
- introducing, under stirring, sodium chloride, hydroxymethylglycinate and disodium EDTA until complete dissolution;
- introducing, again under stirring, the liposomes containing jojoba oil;
- measuring the initial pH and introducing lactic acid in varying amounts up to pH 7.

The eye spray according to the invention is thus an aqueous solution of liposomes containing jojoba, in which the liposomes constitute 0.17 - 2.44% (for example 1.3%) w/v and the aqueous solution constitutes 97.56% - 99.83% (for example 98.7%) in w/v.

### Example 1

For example, to obtain 130 kg of product:
- introduce 115 kg of distilled water into the mixer;
- introduce, under stirring, 1 kg of sodium chloride, 168 g of sodium hydroxymethylglycinate and 129 g of disodium EDTA and leave in recirculation for 30 minutes until complete dissolution;
- introduce, again under stirring, 12.9 kg of liposomes containing jojoba oil;
- measure the initial pH and introduce lactic acid in varying amounts up to pH 7;
- bottle the product, under stirring, into 15 ml bottles and apply the appropriate spray dispenser. Given a density equal to 1, about 8,500 packages are thus obtained.

The end solution presents itself as an opaque, light yellow liquid.

The spray is then simply applied by spraying onto closed eyelids from a distance of 10-15 cm.

It has been shown, by marking with fluorescein, that if applied to the eye area, a spray containing liposomes is capable of depositing the latter on the tear film and is thus a valid alternative to tear substitutes.

## Claims

1. An ophthalmic spray composition comprising liposomes containing jojoba oil and water.

2. A composition according to claim 1, wherein said liposomes consist of (percentage expressed by weight on the liposome weight):
| | |
|---|---|
| Jojoba oil | 0.1 - 2%; |
| Butylene glycol | 0.25 - 2.75%; |
| Phospholipids | 0.04 - 0.44%; |
| Carrageenans | 0.0005 - 0.0055%; |
| Carbomer | 0.0028 - 0.0308%; |
| Sodium chloride | 0.75 - 0.85%; |
| Sodium hydroxymethylglycinate | 0.10 - 0.12%; |
| Disodium EDTA | 0.08 - 0.12; |
| Lactic acid | q.s. at pH = 6.5 - 7.5; |
| Water | q.s. at 100% w/v |

3. A composition according to claim 2, wherein said liposomes consist of:
| | |
|---|---|
| jojoba oil | 0.2%; |
| butylene glycol | 0.5%; |
| phospholipids | 0.08%; |
| carrageenans | 0.001 %; |
| carbomer | 0.0056%; |
| sodium chloride | 0.8%; |
| sodium hydroxymethylglycinate | 0.11%; |
| disodium EDTA | 0.1%; |
| lactic acid | q.s. at pH = 7; |
| water | q.s. at 100% w/v |

4. A process for preparing liposomes according to claims 2-3, wherein the phospholipids are hydrated, the liposomes are sized, the non encapsulated material is removed.

5. A composition according to claims 1-3, wherein said spray consists of: liposomes according to claims 3 and 4 and aqueous solution in a percentage of: 0,17 - 2,44 and 99,83 - 97,56 respectively.

6. A process for preparing a spray according to claim 5, wherein:
- water is introduced into a blender;
- sodium chloride, sodium hydroxymethylglycinate and disodium EDTA are introduced under stirring;
- the liposomes containing jojoba oil are introduced, again under stirring;
- the initial pH is measured and acid is added up to pH 7.

7. An ophthalmic composition according to claims 1-3 and 5 for treating MGD.
